Europäisches Patentamt

European Patent Office

Office européen des brevets

(11) Publication number: **0 242 474 B1**

## EUROPEAN PATENT SPECIFICATION

(45) Date of publication of patent specification: **25.05.94**

(51) Int. Cl.[5]: **C12N  15/01**, A01N 63/00, C05F 11/08, C12N 1/20, C12Q 1/04, //(C12N1/20, C12R1:41)

(21) Application number: **86307416.7**

(22) Date of filing: **26.09.86**

(54) **Rhizobium inoculants.**

(30) Priority: **21.04.86 US 856860**

(43) Date of publication of application:
**28.10.87 Bulletin  87/44**

(45) Publication of the grant of the patent:
**25.05.94 Bulletin  94/21**

(84) Designated Contracting States:
**AT BE CH DE FR GB IT LI LU NL SE**

(56) References cited:
**EP-A- 0 245 931**
**DE-C- 749 153**
**US-A- 4 136 486**

**BIOLOGICAL ABSTRACTS, vol. 71, 1981, abstract no. 42085, Biological Abstracts, Inc., Philadelphia, US; K.D. NOEL et al.: "Diversity and dynamics of indigenous Rhizobium Japonicum populations"**

(73) Proprietor: **W.R. Grace & Co.-Conn.**
**Grace Plaza,**
**1114 Avenue of the Americas**
**New York, New York 10036-7794(US)**

(72) Inventor: **Paau, Alan**
**5404 Jonquil Court**
**Middleton Wisconsin 53562(US)**
Inventor: **Brill, Winston J.**
**4234 Cherokee Drive**
**Madison Wisconsin 53711(US)**

(74) Representative: **Bentham, Stephen et al**
**J.A. KEMP & CO.**
**14 South Square**
**Gray's Inn**
**London WC1R 5LX (GB)**

CHEMICAL ABSTRACTS, vol. 95, 1981, page 347, abstract no. 21099r, Columbus, Ohio, US; J. PLAZINSKI: "Isolation of ineffective and high-effective mutant strains of Rhizobium species using translocatable drug-resistance elements as mutagens"

THE EMBO JOURNAL, vol. 4, no. 13A, 1985, pages 3369-3373, IRL Press Ltd, Oxford, GB; L. ROSSEN et al.: "The nodD gene of Rhizobium leguminosarum is autoregulatory and in the presence of plant exudate induces the nodA,B,C genes"

CHEMICAL ABSTRACTS, vol. 89, 1978, page 320, abstract no. 126212b, Columbus, Ohio, US; R.J. MAIER et al.: "Mutant strains of Rhizobium japonicum with increased ability to fix nitrogen for soybean"

CHEMICAL ABSTRACTS, vol. 105, 1986, pages 404-405, abstract no. 222244h, Columbus, Ohio, US; W.J. BRILL: "Agricultural opportunities from nitrogen fixation research"

CHEMICAL ABSTRACTS, vol. 105, 1986, page 400, abstract no. 187406p, Columbus, Ohio, US; D.V. TARKASHVILI et al.: "Symbiotic properties of nodular soybean bacterial mutants induced by chemical mutagens"

CHEMICAL ABSTRACTS, vol. 97, 1982, page 427, abstract no. 88518m, Columbus, Ohio, US; J. PLAZINSKI: "Tn5-inherited mutant strain of Rhizobium meliloti with a highly increased ability to fix nitrogen for lucerne"

BIOTECHNOLOGY, February 1985, pages 143-149; E.R. OLSON et al.: "Identification of genes involved in the Rhizobium-legume symbiosis by mu-di (KAN, LAC)-generated transcription fusions"

BIOLOGICAL ABSTRACTS/RRM, abstract no 29076465, Department Soil Science, Minnesota, US; F.M. ROBERT et al.: "Response of 3 indigenous serogroups of Rhizobium-Japonicum to the rhizosphere of preemergent seedlings of soybean"

**Description**

The present invention relates to a method for creating novel Rhizobium strains which are both biologically competitive and which have high nitrogen fixation ability and to specific novel Rhizobium strains.

The phenomenon of the symbiotic relationship between legumes and the Rhizobium bacteria which nodulate their roots is widely known. The bacteria, living in the nodules in the legume roots, fix nitrogen directly from the atmospheric nitrogen thereby converting nitrogen to biologically useful nitrogenous compounds. This process not only provides nitrogen to the plant for protein synthesis, but also enriches the soil in which the legume is grown by leaving nitrogenous nutrients in the soil for later crops. Examples of legumes which are capable of symbiotic relationship with Rhizobium bacteria are peas, beans, alfalfa, red clover, white clover, vetch, and lupines.

Since other agriculturally important plants are unable, either directly or indirectly, to fix nitrogen directly from the air, and thus generally are dependent upon nitrogenous fertilizers to introduce sufficient nitrogen content in the soil for good yield, it is quite common for legumes to be alternated each growing season with non-legume crops in important agricultural areas to facilitate economical enhancement of nitrogen content in the soil. This practice, known as crop rotation, is wide spread. Furthermore, some legumes, and in particular soybeans, peas, beans, and alfalfa, are important commercial crops in their own right and growth of these crop plants is greatly facilitated by an ample supply of combined or fixed nitrogen.

There are a number of species of Rhizobium bacteria and different Rhizobium species are adapted to form symbiotic relationships, and nodules, only in the roots of specific legumes. For example, R. japonicum nodulates the roots of soybeans, R. trifolii nodulates the roots of clovers, R. meliloti grows symbiotically with alfalfa and sweet clovers, R. leguminosarum is used with peas and vetches, and R. phaseoli nodulates the roots of common garden beans. Therefore, in commercial agricultural practices, it is quite a common practice to inoculate the soil or the seeds of the legumes with a culture of the appropriate Rhizobium species. This inoculant is commonly done by coating the seeds before planting, dusting planted seeds, or by spreading the inoculant in the furrows of the planted legume seeds.

In creating and formulating a Rhizobium inoculant, it is desirable that the Rhizobium strain contained in the inoculant have an enhanced ability to fix nitrogen for the benefit of the plant and for soil conditioning. Therefore, much research has logically been conducted on methods for improving the nitrogen fixation of Rhizobium species. Most of the currently commercially available Rhizobium inoculants include strains having high nitrogen fixing ability selected from natural populations.

However, the ability to fix nitrogen does not ensure the survivability of the bacteria in field conditions. Because legumes have been cultivated for many generations in most of the important agricultural areas of the world, many strains of Rhizobium bacteria now freely live indigenously in important agricultural areas. The bacteria may not have been naturally indigenous to all these areas, but introduced strains have populated many of these areas and since they survive freely they may be considered effectively indigenous.

All free-living bacteria, in the soil or elsewhere, are continually subject to environmental pressures, and naturally tend to mutate and be selected by the pressures of the ecological niche which they occupy to be further adapted and competitive for that niche. The indigenous Rhizobium strains are, of course, subject to these pressures. Therefore many of the soils in agricultural areas now contain indigenous Rhizobium strains which have evolved to be adapted for survival in competitive existence in that particular soil environment. Accordingly, even if a farmer introduces into a field a Rhizobium inoculant strain having high nitrogen fixing capability, there is no guarantee that the introduced Rhizobium strain will predominate the roots of the legume plants. The introduced inoculant strain promptly enters into ecological competition with the indigenous strains already present in the soil to nodulate the plants, and, many times, the indigenous populations are an advantage because they are previously selected by ecological pressures for competition in precisely the environment of that field or climatic area. Accordingly, even a high nitrogen fixing Rhizobium strain which can effectively nodulate the legume and fix nitrogen effectively without competition may be an ineffective inoculant in the field since the introduced strain may not actually populate the roots of the legumes, but may be overwhelmed by a locally indigenous strain, which may not have optimal nitrogen fixation abilities. No prior teachings are known on either the desirability of or the process for creating novel Rhizobium strains which are both competitive in field conditions and have high nitrogen fixation abilities.

It has been known previously that legume root exudate can be used to cause Rhizobium cells to develop more lectin-binding sites. It is also known that such exudates can be used as a pretreatment to foster and to enhance the ability of Rhizobium to nodulate when inoculated onto a legume plant.

The present invention provides a method for creating a novel strain of Rhizobium bacteria useful as a crop inoculant for legume species in a selected geographic area characterised in that the method comprises the steps of: isolating a number of Rhizobium cultures from the geographic area; identifying the naturally predominant Rhizobium strain in the geographic area by comparative analysis of the cultures; verifying the competitiveness of the identified naturally predominant strain by conducting a field trial of the strain and measuring the recoverability of that strain from nodules in plants in the trial field; exposing a culture of the naturally predominant strain to a mutagenic agent; and selecting among the mutagenized bacteria for mutants having high nitrogen fixation ability in association with the legumes.

The present invention is also directed toward the novel strains identified as ATCC 53535 and 53536 which are created through the use of this process.

It is a feature of the present invention in that it is able to select for strain competitiveness and prevalence in particular agricultural environmental conditions without the necessity for simulating the competitive conditions on a bacterial strain in an artificially controlled environment, a simulation which has proven extraordinarily difficult to perfect in practice.

An embodiment of the present invention will now be more particularly described by way of example and with reference to the accompanying drawings, in which:

FIGURE 1 is a flow chart of the methodology used to generate high nitrogen fixing strains of the competitive Rhizobium strains isolated and identified in accordance with the procedure of the present invention;

FIGURE 2 is a chart illustrating the enhancement of nitrogen fixation activity obtained from one mutagenized naturally predominant strain;

FIGURE 3 is a chart of the enhanced nitrogen fixing activity obtained from a second strain; and

FIGURE 4 is a chart illustrating enhancement of nodulation obtained by inoculant pre-treatment.

The practice of the process of creating novel Rhizobium strains pursuant to the present invention proceeds through a series of steps. First, it is necessary to isolate a number of Rhizobium cultures from a selected geographic area which are then analysed to identify naturally predominant strains of Rhizobium. The identified naturally predominant strains are then subject to mutagenesis with the mutagenized colonies being selected for high nitrogen fixation. After high nitrogen fixing mutants are discovered, the mutants are returned to field test trial to determine that they both remain competitive and remain sufficiently high nitrogen fixing in the field to increase yield. To fully understand the details of each of these procedures, they will each be discussed in order.

The process of isolation of indigenous Rhizobium cultures to begin with the identification of competitive strains. Contrary to prior strategies for creating Rhizobium inoculants, no effort is taken in accordance with the present invention to try to select for, or engineer for, competitiveness in actual field condition. Instead, the first step is directed toward identifying those indigenous naturally predominant strains which are already present in the selected geographic growing area for the legume crop selected. For example, selecting soybean as a legume, one would select the locales in which soybeans are grown, and identify the strains of indigenous Rhizobium japonicum naturally predominant in that locale. As used herein, the term "indigenous" does not mean naturally occurring before the advent of mankind, since R japonicum, for example, is an introduced species in the Western Hemisphere, but refers to the strains which naturally thrive in the soils in the agricultural areas at present. Most soils, for example, in soybean producing areas of the United States now have indigenous local strains of R japonicum naturally present and the strains have evolved to the soil types and climatic regions of the country.

The determination and identification of the competitive strains proceeds first by the large scale collection of nodules from the legume cultivation region which has been selected. Legume crop fields are located which have not been inoculated with any Rhizobium inoculant. Plants are removed from the fields, and the nodules are harvested from the roots of plants grown in those fields. The nodules are surface-sterilized and then crushed so that the Rhizobium colonies therein can be accessed. Cells from the crushed nodules are plated onto suitable growth media, i.e. nutrient agar medium, and are cultivated in the laboratory. These strains can then proceed through the strain identification process.

Identification of bacterial strains is an evolving technology at present. At present, most strains of bacteria are identified and classified based on the results of a series of tests. The tests include examinations of morphology, bacterial serology, quantitative antibiotic sensitivity of the various strains, phage sensitivity of the strains, and the protein pattern created by a strain by one or two dimensional polyacrylamide gel electrophoresis. Any or all of these techniques may be used together to identify the strains. The most advantageous technique used by the applicants here, and considered the best combination of ease and effectiveness, is one-dimensional gel electrophoresis.

4

Preferably, large numbers of nodules are collected and large numbers of Rhizobium cultures are obtained from the particular locale for the crop in question. For example, it is appropriate to obtain cultures for hundreds, if not thousands, of Rhizobium strains from a given geographic area, and a given crop such as soybean. These thousands of strains are then analyzed by gel electrophoresis. The electrophoresis gel patterns are then analyzed. Those patterns which predominate in the growing areas are defined, for purposes of this procedure, to characterized the strains which are naturally predominant. In other words, the large numbers of gel plates are analyzed and placed into groups reflecting the groupings of the gel patterns. The numbers of cultures falling in each group are then compared to determine which of the groupings, or strains, is most represented and therefore predominates in the particular region under consideration. Those isolates which are found to have identical gel patterns are defined to be the same strain. It will be discovered that different frequently isolated strains will be found to be differentially competitive in different growing regions, and that certain strains will seem to predominate in any one localized growing region.

Once the most widely isolated strain in a given growing region is determined, that strain is considered the naturally predominant and competitive strain for purposes of this procedure. In order to fully verify its competitiveness, and to ensure that the strain has not previously mutated while in culture, it is then appropriate to conduct a field test of the strain to ensure that it nodulates properly and that its competitive is replicatable in the actual field environment. Also appropriate is a test for nitrogen fixing activity. This step can be performed in green house or phytotron on individual plantlets in pots or trays. The plants are inoculated with the putative predominant competitive strain and then are assayed for acetylene reduction. This not only verifies that the nitrogen fixing activity of the strain occurs in conjunction with the selected crop plant, but serves as a base line for later efforts to select for increased nitrogen fixing activity.

The confirmation test for competitiveness is a field test for nodulation activity. This is done by planting fields of the putative naturally predominant competitive strain. Controls should include both uninoculated seeds and plants inoculated with commercial, but presumably less-competitive, strains. In order to be deemed a successful naturally predominant competitive strain, the selected strain should be recoverable from nodules in inoculated plants at a rate significantly higher than recoverable from plants which were either not inoculated or inoculated with non-competitive strains and also at a rate significantly higher than the commercial strain can be recovered from nodules..

Strains which can be re-isolated at high frequency from nodules of legume plants inoculated with the strain of interest are determined for purposes of experiment to be naturally predominant and competitive under field conditions. Thus at this stage of the procedure strains have been isolated and identified which are indigenous, but which can also withstand laboratory isolation and be reintroduced while retaining their competitiveness. In addition, since by this step we have verified that the nodulation of the plants is increased by inoculation of this strain, it is also demonstrated that increased nodulation can be obtained through the use of this strain, even without enhanced nitrogen fixing activity.

The next step is to proceed to mutagenize cultures of the naturally dominant strains. Cultures of the strains are exposed to a mutagenic agent, such as radiation or a chemical agent. It is preferred within the present experiment that the mutagen used is the chemical mutagen nitrosoguanidine. The bacteria exposed to the mutagen are transferred and allowed to grow out several times in a liquid medium. The mutagenized cultures are then spread on agar plates to obtain individual colonies. Once individual colonies are established, the colonies are again transferred to a liquid medium and allowed to grow out. Samples of each of the grown out colonies can then be inoculated into a serum vial containing a seedling of the legume plant to which the Rhizobium is associated. The seedling is isolated from the environment, such as by covering with a sterile transparent container, and then is grown under conditions fostering the growth of the legume plant. After a period of time, an assay for nitrogen fixing activity, again accomplished by testing for acetylene reduction activity of the seedlings by gas chromatography or other similar process is performed. By comparing the comparative nitrogen fixing activity of the mutated bacteria with the base line for the naturally predominant strain, mutagenized progeny which have enhanced nitrogen fixing activity can be isolated. The strains having the most promising nitrogen fixing activity can then be returned to culture and grown out to provide sufficient quantities for additional field testing.

Once high nitrogen fixing candidate mutant strains have been identified, those strains must then be analyzed again to ensure that the natural competitiveness has not been lost through the mutation process. Accordingly, all the candidate strains must again be grown out and must be individually tested for competitiveness in actual field trials. Concurrently, nitrogen fixing trials can be replicated in greenhouse or phytotron to ensure repeatable and reliable enhanced nitrogen fixation capability. Competitiveness can only be truly assessed in field trials because of the state of the art of the technology of replicating competitive field conditions in the greenhouse or phytotron. Thus the strains are then grown out and inoculated into the appropriate legume plants in the crop growing areas. Then the populations of the nodules in the plants can be examined midseason to determine which strains of bacteria are populating those nodules. For those strains which remain competitive and achieve enhanced nitrogen fixing activity, those strains should be present in the nodules in numbers quantitatively increased over non-competitive strains. In addition, fields containing the mutant strains having high nitrogen fixation and competitive advantages should give enhanced yield over those fields having no inoculant or other commercial, non-competitive strain, inoculants available.

It was decided to generate novel strains of Rhizobium japonicum bacteria for particular uses as a soybean inoculant in two major soybean-producing areas of the United States. As a first step in this process, an effort was undertaken to identify the naturally predominant strains of R. japonicum in the selected states of Iowa and Louisania. Field teams visited soybean fields in each state in which no commercial inoculant was applied to the fields during planting. Nodules were recovered from soybean plants in the fields. The nodules were surface sterilized and crushed. Rhizobium cells isolated from the nodules were then plated on to a nutrient agar medium, in which they were cultured and returned to the laboratory.

In the laboratory, cultures of the strains were grown up and then analyzed using one-dimensional gel polyacrylamide gel eletrophoresis. Protein gel patterns were created for each of the Rhizobium isolates obtained from each state. The gel patterns from the isolates were then comparatively analyzed to determine which patterns were predominant in each of the two designated states. That analysis was able to identify certain gel patterns which predominated from the samples taken. The gel patterns were grouped and assigned tentative strain numbers, numbers assigned to the groups of gel patterns to help group the patterns into related strains.

From the Iowa samples, the strain designated IA2838 was identified, along with one other Iowa strain, as the most predominant. From the Louisiana samples, a strain designated LA1304 was determined, again along with one other strain, to be the strain best represented among the gel patterns. For purposes of these procedures, both the strains were determined to be naturally predominant strains.

In order to verify the infective activity of these Rhizobium strains, a phytotron test of infectivity of each of these Rhizobium strains was conducted. Bacteria from each strain was inoculated onto soybean seeds grown up individually in the phytotron. Acetylene reduction assays were taken on the young soybean plants to indicate relative nitrogen fixing activity of the strain when inoculated into the soybeans. Both of these strains were found to be infective in soybean and to form nodules in the roots of soybean plants, and to exhibit reasonable, though not enhanced, acetylene reduction activity indicating nitrogen fixation.

To verify that these strains were indeed predominant and competitive in the locales from which they were isolated, a field test was conducted using the naturally predominant strains in actual field conditions. The strains were used as an inoculant during planting under normal local soybean growing practices in soybean fields in both Iowa and Louisiana. To investigate the differential competitiveness on the Iowa and Louisiana strains, naturally competitive strains from both locales were inoculated into fields in both areas. In the field trials in each state, there were double controls, one control being plants which were not inoculated intentionally with any inoculant, and the second control being planted with a commercially available inoculant from Nitragin Co. In the Iowa site, the Iowa naturally predominant strains were identified in 37% of the total Rhizobial population recovered from nodules of uninoculated plants. This indicates a population of this naturally occurring strain sufficient to cause nodulation in plants even without intentional inoculation. Where the commercial inoculant was used, bacteria corresponding to the experimental Iowa naturally predominant strain were found in 30% of the total Rhizobial population in nodules recovered. For the plants in which the experimental inoculant was utilized, 67% of the Rhizobial population in nodules were found to be the Iowa strains of naturally predominant bacteria. By contrast, only 5% of the Rhizobial population in nodules were found to be bacteria corresponding to the Louisiana naturally predominant strains, which were also included in the experimental inoculant. Similar results were obtain in the Louisiana site where the uninoculated plants had nodules which were occupied at 33% by the Louisiana naturally predominant strains, the Nitragin Co. inoculated plants yielded the naturally predominant strains in 13% of the Rhizobial population in their nodules, and the experimentally inoculated plants had the naturally predominant strains in 63% of the Rhizobial population in their nodules. The experimental inoculant again included populations of both naturally predominant strains. At the Iowa site, only about 5% of the Rhizobial population nodules contained bacteria of the naturally predominant Louisiana strain, with a similar result being reached for the naturally predominant Iowa strains at the Louisiana site. Thus it was determined that the naturally predominant strains effectively nodulated plants in field conditions and were very competitive with existing indigenous Rhizobium and with other inoculants to compete for nodulation sites in soybean roots. In addition, it was also made clear by this test that for each of the two geographic locales selected, the naturally predominant strains from those locales were superior in each locale to the naturally predominant strain from the other.

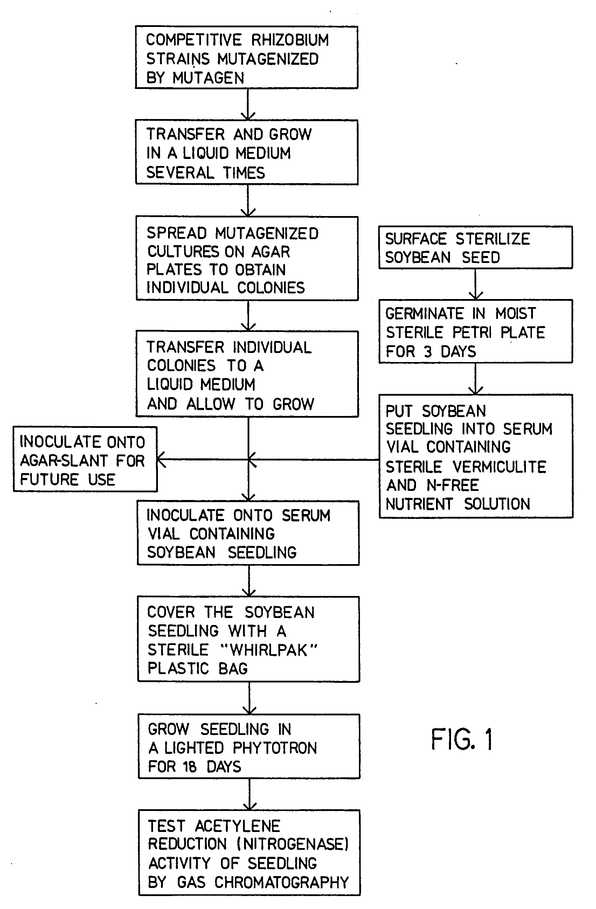

Once the competitiveness and nodulation activity of the naturally predominant strains was verified, the strains were then mutagenized and selected for high nitrogen fixation activity. Shown at Figure 1 is a flow chart of the process used to perform this step. The chemical mutagen used was nitrosoguanidine. The bacteria were exposed to the mutagen and then transferred and allowed to grow in a liquid medium several times. The mutagenized colonies were then spread on agar plates to obtain individual colonies which were again transferred to a liquid medium to grow out. Bacteria from the grown out colonies were placed on soybean seedlings grown out from surfaced sterilized soybean seed grown in a moist sterile petri plate for three days. The soybean seedling and the inoculant was placed in a serum vial containing sterile vermiculite and nitrogen free nutrient solution. The soybean seedling was covered with a sterile plastic bag and grown in a lighted phytotron for 18 days, after which the acetylene reduction activity was determined by gas chromatography. Mutagenized colonies which showed the best nitrogen fixing activity (acetylene reduction activity) were re-inoculated into additional plants and retested to verify that the activity was replicatable.

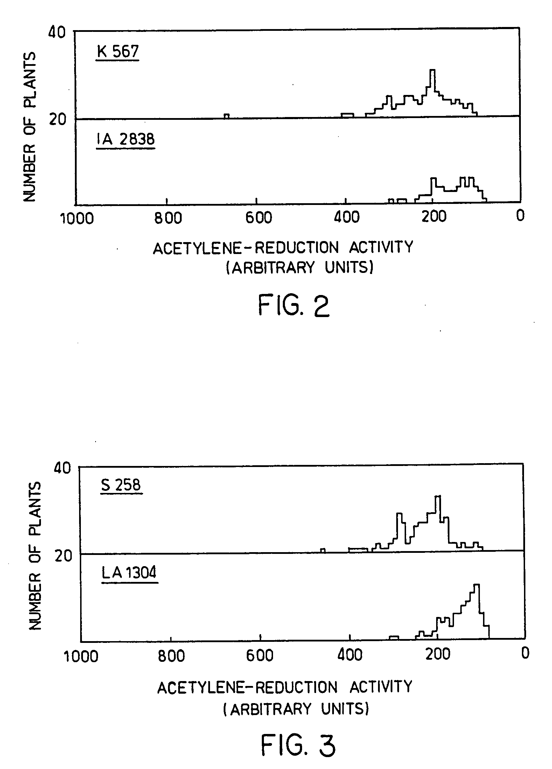

Shown in Figures 2 and 3 are charts illustrating the enhancement of nitrogen fixation activity obtained from the mutagenized strains. Illustrated in Figure 2 demonstrates the relative acetylene reduction activity of strain IA2838 compared to Iowa strain K567, which a mutagenized version of IA2838. The units are arbitrary. Shown in Figure 3 is a similar chart showing the enhanced nitrogen fixation activity of strain S258 which is a high-nitrogen fixing mutant of naturally competitive strain Louisiana LA1304.

The mutant strains S258 and K567 were then used as inoculants in actual field tests to attempt to verify competitiveness and increased nitrogen fixation activity by measuring yield. Each strain was tested against its wild type ancestor. Strain S258 inoculated using a peat formulation exhibited a 9.9% increase in yield over its wild type ancestor used similarly as an inoculant in Louisiana on soybean of variety Centennial. On a similar test utilizing a peat formulation grown in Illinois on Williams 82 soybeans, no difference in yield was obtained between the mutant strain and the wild type. Strain K567 inoculated using a peat formulation, as compared to strain IA2838, also inoculated using a peat formulation, on fields of Corsoy 79 and Williams 82 soybeans in fields in Ohio and Wisconsin exhibited an average yield increase of 13.4%. Field trials of both of these strains were conducted using uninoculated fields and also against using an existing commercial inoculant (Nitragin), and both mutated strains showed statistically significant yield enhancement over both these controls also.

It is also possible to further enhance the nodulation competitiveness of these competitive mutant strains. If the speed with which the Rhizobium begin the nodulation process can be increased, the first nodulating bacteria will have a competitive advantage, because they already occupy the nodules, over other strains. It is already known that soybean root extract can be used to enhance the nodulation activity of Rhizobium japonicum strains. This phenomenon also is effective with the novel strains produced by the present process.

To verify that effect, soybean seeds were germinated in soil contained in clear plastic containers. The seeds were sprouted and, after sprouting, a mark was made on the container indicating the position of the root tip. The soil was then inoculated with a viable culture of Rhizobium japonicum strain K567. The plants were allowed to grow out and then the nodules in the roots of the plants were scored, both for number and for location relative to the root tip marker. Control plants were inoculated with untreated K567 bacteria while experimental plants were inoculated with bacteria pre-treated with a nutrient broth in which soybean seedlings had been previously grown.

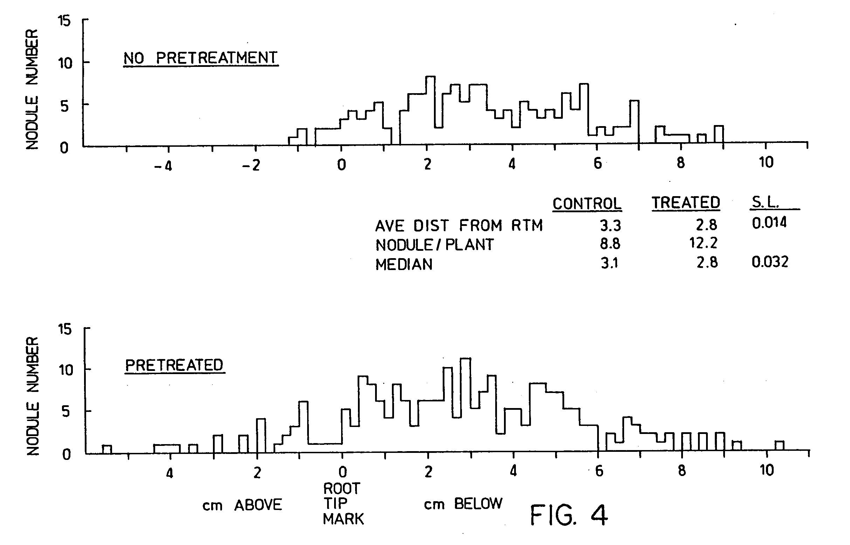

The results are displayed in Fig. 4 and Table 1 below. Fig. 4 indicates that there were elevated numbers of nodules overall in the experimental plants and that the nodules were generally higher on the plant roots, indicating faster nodule formation. Table 1 verifies this result. Therefore, this pre-treatment phenomenon is effective even for the novel competitive strains created by this process.

## Table 1

|  | Control | Pre-Treated |
|---|---|---|
| Average distribution of the Uppermost nodule from the root tip marker (rtm) (minus indicates above the r.t.m.) | -0.02cm | -0.49cm |
| Percent of nodulation below the r.t.m. | 82.4% | 84.5% |
| Percent of nodulation on lateral roots | 32.0% | 4.9% |
| Nodules per plat on primary root | 1.9 | 8.3 |
| Nodules per plant on lateral root | 2.7 | 1.5 |

Thus this example demonstrates that novel competitive and high nitrogen fixing strains of Rhizobium bacteria can be created using the process of the present invention. This approach has two advantages over approaches previously known in the technology. This approach manages to select competitive Rhizobium bacteria without having to attempt to mimic competitive any field conditions in order to select for strains that would be competitive in the field. Previous inoculant strains have not been properly competitive, and the state of the art in mimicing field conditions to accurately select for competitiveness is not very sophisticated. In addition, this approach does not require that the experimenter identify, quantify, or even study which characteristics of the Rhizobium itself are necessary or advantageous in making the strain competitive in the field (ie. able to become a major occupant in nodules of soybeans or other legumes in field crop conditions). This process takes advantage of the natural adaptation of Rhizobium activity occurring in agricultural fields in any event.

As well as the mutagenized species S258 and K567, the selected species IA2838 and LA1304 are also considered novel when used as a plant inoculant.

The four strains described herein have been deposited in the Cetus Master Culture Collection and the American Type Culture Collection, this latter on 10th September 1986, and are identified as follows:

| Strain | CMCC No. | ATCC No. |
|---|---|---|
| IA2838 | 1885 | 53538 |
| LA1304 | 1886 | 53537 |
| S258 | 2847 | 53536 |
| K567 | 2848 | 53535 |

**Claims**

1. A method for creating a novel strain of Rhizobium bacteria useful as a crop inoculant for a legume species in a selected geographic area, characterised in that the method comprises the steps of: isolating a number of Rhizobium cultures from the geographic area; identifying the naturally predominant Rhizobium strain in the geographic area by comparative analysis of the cultures; verifying the competitiveness of the identified naturally predominant strain by conducting a field trial of the strain and measuring the recoverability of the strain from nodules in plants in the trial field; exposing a culture of the naturally predominant strain to a mutagenic agent; and selecting among the mutagenized bacteria for high nitrogen fixation activity in association with the legume.

**2.** A method as claimed in claim 1, characterised in that the step of identifying the naturally predominant strain includes the step of comparatively analysing the constituent proteins of the bacteria in each isolated culture.

**3.** A method as claimed in claim 2, characterised in that the step of identifying the naturally predominant strain is performed by one-dimensional polyacrylamide gel electrophoresis.

**4.** A method as claimed in any one of the preceding claims, characterised by a further step of verifying the competitiveness of the mutagenized bacteria by field testing the mutagenized strain and evaluating the yield enhancement created by it.

**5.** A method as claimed in any one of the preceding claims, characterised in that nitrogen fixation activity is measured by assays of acetylene reduction.

**6.** A method as claimed in any one of the preceding claims characterised in that the Rhizobium is R.japonicum, R.trifolii, R.leguminosarium, R.meliloti, R. lupini, or R. phaseoli.

**7.** A method as claimed in claim 6, characterised in that the Rhizobium is of species R.japonicum.

**8.** Rhizobium bacteria identified as ATCC53536.

**9.** Rhizobium bacteria identified as ATCC53535.

**Patentansprüche**

**1.** Verfahren zur Herstellung eines neuen Rhizobium-Bakterienstammes, der als Ernte-Inokulum für eine Leguminosen-Spezies in einem ausgewählten geographischen Gebiet brauchbar ist, dadurch gekennzeichnet, daß das Verfahren die Schritte umfaßt:
Isolieren einer Anzahl Rhizobium-Kulturen aus dem geographischen Gebiet;
Identifizieren des natürlicherweise prädominanten Rhizobium-Stammes in dem geographischen Gebiet durch vergleichende Analyse der Kulturen;
Prüfen der Konkurrenzfähigkeit des identifizierten, natürlicherweise prädominanten Stammes durch Ausführung eines Feldversuches mit dem Stamm und Messung der Wiedergewinnbarkeit des Stammes aus den Knötchen in den Pflanzen des Testfeldes; Behandeln einer Kultur des natürlicherweise prädominanten Stammes mit einem mutagenen Agens; und
Selektieren der mutagenisierten Bakterien im Hinblick auf hohe Stickstoff-Fixierungsaktivität in Verbindung mit der Hülsenfrucht.

**2.** Verfahren nach Anspruch 1, dadurch gekennzeichnet, daß der Schritt des Identifizierens des natürlicherweise prädominanten Stammes den Schritt des vergleichenden Analysierens der Protein-Bestandteile der Bakterien in jeder isolierten Kultur einschließt.

**3.** Verfahren nach Anspruch 2, dadurch gekennzeichnet, daß der Schritt des Identifizierens des natürlicherweise prädominanten Stammes durch eindimensionale Polyacrylamid-Gelelektrophorese ausgeführt wird.

**4.** Verfahren nach irgendeinem der vorangehenden Ansprüche, gekennzeichnet durch einen weiteren Schritt, in welchem die Konkurrenzfähigkeit der mutagenisierten Bakterien in einem mit dem mutagenisierten Stamm durchgeführten Feldversuch getestet wird und die so verursachte Ertrags-Steigerung bewertet wird.

**5.** Verfahren nach irgendeinem der vorangehenden Ansprüche, dadurch charakterisiert, daß die Stickstoff-Fixierungsaktivität durch Acetylen-Reduktions-Tests gemessen wird.

**6.** Verfahren nach irgendeinem der vorangehenden Ansprüche, dadurch gekennzeichnet, daß das Rhizobium R. japonicum, R. trifolii, R. leguminosarium, R. meliloti, R. lupini oder R. phaseoli ist.

**7.** Verfahren nach Anspruch 6, dadurch gekennzeichnet, daß das Rhizobium der Spezies R. japonicum angehört.

**8.** Rhizobium-Bakterien, identifiziert als ATCC53536.

**9.** Rhizobium-Bakterien, identifiziert als ATCC53535.

**Revendications**

**1.** Méthode de création d'une nouvelle souche de bactéries de Rhizobium, utile comme inoculant de la récolte pour une espèce de légume dans une zone géographique choisie, caractérisée en ce que la méthode comprend les étapes de : isoler un certain nombre de cultures de Rhizobium de la zone géographique; identifier la souche naturellement prédominante de Rhizobium dans la zone géographique par analyse de comparaison des cultures; vérifier la compétitivité de la souche naturellement prédominane identifiée en entreprenant un essai en champ de la souche et en mesurant la récupérabilité de la souche des nodules dans les plantes du champ essayé; exposer une culture de la souche naturellement prédominante à un agent de mutagénèse; et sélectionner, parmi les bactéries mutagénisées, la grande activité de fixation de l'azote en association avec le légume.

**2.** Méthode selon la revendication 1, caractérisée en ce que l'étape d'identifier la souche naturellement prédominante comprend l'étape d'analyser par comparaison les protéines constituant la bactérie dans chaque culture isolée.

**3.** Méthode selon la revendication 2, caractérisée en ce que l'étape d'identifier la souche naturellement prédominante est accomplie par une électrophorèse sur gel de polyacrylamid sur une dimension.

**4.** Méthode selon l'une quelconque des revendications précédentes, caractérisée par une autre étape de vérification de la compétitivité de la bactérie ayant subi la mutagénèse par des essais en champ de la souche mutagénisée et évaluation de l'amélioration du rendement ainsi créée.

**5.** Méthode selon l'une quelconque des revendications précédentes, caractérisée en ce que l'activité de fixation de l'azote est mesurée par des déterminations de la réduction de l'acétylène.

**6.** Méthode selon l'une quelconque des revendications précédentes, caractérisée en ce que Rhizobium est R. japonicum, R. trifolii, R. leguminosarium, R. meliloti, R. lupini ou R. phaseoli.

**7.** Méthode selon la revendication 6, caractérisée en ce que le Rhizobium est de l'espèce R. japonicum.

**8.** Bactérie de Rhizobium identifiée par ATCC53536.

**9.** Bactérie de Rhizobium identifiée par ATCC53535.

COMPETITIVE RHIZOBIUM STRAINS MUTAGENIZED BY MUTAGEN

↓

TRANSFER AND GROW IN A LIQUID MEDIUM SEVERAL TIMES

↓

SPREAD MUTAGENIZED CULTURES ON AGAR PLATES TO OBTAIN INDIVIDUAL COLONIES

↓

TRANSFER INDIVIDUAL COLONIES TO A LIQUID MEDIUM AND ALLOW TO GROW

INOCULATE ONTO AGAR-SLANT FOR FUTURE USE

↓

INOCULATE ONTO SERUM VIAL CONTAINING SOYBEAN SEEDLING

↓

COVER THE SOYBEAN SEEDLING WITH A STERILE "WHIRLPAK" PLASTIC BAG

↓

GROW SEEDLING IN A LIGHTED PHYTOTRON FOR 18 DAYS

↓

TEST ACETYLENE REDUCTION (NITROGENASE) ACTIVITY OF SEEDLING BY GAS CHROMATOGRAPHY

SURFACE STERILIZE SOYBEAN SEED

↓

GERMINATE IN MOIST STERILE PETRI PLATE FOR 3 DAYS

↓

PUT SOYBEAN SEEDLING INTO SERUM VIAL CONTAINING STERILE VERMICULITE AND N-FREE NUTRIENT SOLUTION

FIG. 1

11

FIG. 2

FIG. 3

FIG. 4